# EUROPÄISCHE PATENTANMELDUNG

(11) **EP 1 013 900 A1**
(43) Veröffentlichungstag der Anmeldung: **28.06.2000**
(21) Anmeldenummer: 99124019.3
(22) Anmeldetag: 08.12.1999
(51) Int. Cl.: F01M 1/18, G01N 27/00

(54) **Verfahren zur Überwachung der ausreichenden Ölschmierung eines Verbrennungsmotors und Verbrennungsmotor zur Durchführung des Verfahrens**

(30) Priorität: 22.12.1998 DE 19859337
(71) Anmelder: Mannesmann VDO Aktiengesellschaft, 60388 Frankfurt am Main (DE)
(72) Erfinder: Kern, Eckhart, 65719 Hofheim (DE); Wallrafen, Werner, 65719 Hofheim (DE); Acht, Joachim, 65936 Frankfurt (DE); Wehrmeyer, Volker, 61462 Königstein (DE)
(74) Vertreter: Rassler, Andrea, Dipl.-Phys.

(57) **Zusammenfassung**

Zur Überwachung der ausreichenden Ölschmierung eines Verbrennungsmotors werden mittels eines in einer Ölleitung (5) einer Druckumlaufschmierung angeordneten, mit einer Auswertelektronik (9) verbundenen Sensors (8) Luftblasen im Ölstrom detektiert. Diese Luftblasen sind ein Frühindikator für eine nicht ausreichende Schmierung des Verbrennungsmotors.

## Beschreibung

Die Erfindung betrifft ein Verfahren zur Überwachung der ausreichenden Ölschmierung eines Verbrennungsmotors, insbesondere eines Fahrzeugs, mittels eines in einer Ölleitung einer Druckumlaufschmierung angeordneten, mit einer Auswertelektronik verbundenen Sensors. Weiterhin betrifft die Erfindung einen Verbrennungsmotor zur Durchführung dieses Verfahrens.

Das ordnungsgemäße Arbeiten der Druckumlaufschmierung ist für die Lebensdauer von Verbrennungsmotoren von entscheidender Bedeutung. Reißt an den Zylinderwänden der Ölfilm ab, kommt es sehr rasch zu einer Überhitzung des Motors, die rasch zu einem ernsten Motorschaden führt. Derzeit wird die ausreichende Ölschmierung von Verbrennungsmotoren durch Sensoren nur indirekt überwacht, welche den Druck in der Ölleitung messen. Fällt der Druck beispielsweise infolge eines Versagens der Ölpumpe oder einer Undichtigkeit im Ölkreislauf ab, dann erhält der Fahrer ein Warnsignal. Zusätzlich überwacht man oftmals mittels eines Sensors den Ölstand in der Ölwanne, um sicherzustellen, dass stets ein ausreichend großer Ölvorrat vorhanden ist.

Es hat sich in der Praxis jedoch gezeigt, dass ein Öldruckabfall oftmals erst festzustellen ist, wenn es bereits zu ernsten Motorschäden infolge nicht ausreichender Schmierung gekommen ist. Auch die Überwachung des Ölstandes in der Ölwanne stellt keine ausreichende Ölversorgung sicher. Durch dynamische Fahrzustände, beispielsweise beim Durchfahren einer längeren Kurve, bei einer Bergfahrt oder während eines Beschleunigungsvorganges, kann sich der Ölspiegel in der Ölwanne so verlagern, dass die Ölpumpe statt Öl Luft ansaugt und dadurch die Schmierung vorübergehend ausfällt und es zu einer nicht ausreichenden Schmierung kommt.

Der Erfindung liegt das Problem zugrunde, ein Verfahren zur Überwachung der ausreichenden Ölschmierung eines Verbrennungsmotors zu entwickeln, durch welches eine möglichst frühzeitige Warnung erfolgen kann, wenn die Gefahr einer nicht ausreichenden Motorschmierung besteht. Weiterhin soll ein Verbrennungsmotor entwickelt werden, welcher nach einem solchen Verfahren arbeitet.

Das erstgenannte Problem wird erfindungsgemäß dadurch gelöst, dass mittels des Sensors und der Auswertelektronik Gasblasen im Ölstrom der Ölleitung ermittelt werden.

Durch ein solches Verfahren kann bereits eine Warnung erzeugt werden, wenn infolge dynamischer Fahrzustände vorübergehend mit dem Öl Luft angesaugt wird oder wenn sehr geringe Undichtigkeiten im Ansaugbereich des Ölkreislaufes auftreten, durch die Luft in das System gelangt. Hierdurch wird schon rechtzeitig die Gefahr einer unzureichenden Motorschmierung erkannt. Ein Warnsignal kann bereits erzeugt werden, bevor die Motorschmierung abreißt. Das erfindungsgemäße Verfahren ist nicht auf die Anwendung bei Kraftfahrzeugen begrenzt. Die Gefahr eines Ansaugens von Luft infolge dynamischer Fahrzustände ist beispielsweise bei Schiffen sehr groß, wenn große Wellen auftreten, oder bei Segelschiffen, die mit Maschine fahren und zugleich unter Segeln stehen, weil durch die Segel oftmals eine starke und lang anhaltende Schräglage auftritt. Auch bei Motorflugzeugen besteht die Gefahr einer unzureichenden Schmiermittelversorgung infolge dynamischer Flugzustände.

Je mehr Abstand der Sensor von den Schmierstellen des Verbrennungsmotors hat, um so früher kann bei Gefahr einer nicht ausreichenden Motorschmierung ein Warnsignal erzeugt werden. Deshalb ist es vorteilhaft, wenn der Sensor in einer Ölansaugleitung in Strömungsrichtung vor einer Ölpumpe der Druckumlaufschmierung angeordnet wird.

Wenn man eine etwas geringere Detektorgeschwindigkeit - d. h. eine geringere Zeitspanne zwischen Detektionszeitpunkt und Ankunft der Blasen in den Zylindern des Motors - hinnehmen will, kann man jedoch auch vorsehen, dass der Sensor in Strömungsrichtung hinter der Ölpumpe und hinter einem der Ölpumpe nachgeschalteten Ölfilter angeordnet wird. Das hat den Vorteil, dass der Sensor auch auf Luftblasen anspricht, welche infolge von Undichtigkeiten des Ölfilters oder beispielsweise der Ölleitung vor dem Sensor in den Ölstrom gelangen.

Der Sensor zum Detektieren von Luftblasen kann sehr unterschiedlich gestaltet sein. Man könnte beispielsweise mit einem optischen Sensor die Lichtdurchlässigkeit des Ölstromes überwachen. Besonders einfach ist der Sensor gestaltet, wenn ein nach dem thermoelektrischen Prinzip arbeitender Sensor verwendet wird. Solche Sensoren sind zur Niveauüberwachung bereits gebräuchlich und deshalb relativ kostengünstig erhältlich. Der Sensor ist von einfachem Aufbau und daher besonders auch für eine Großserienfertigung geeignet, wenn er vorteilhaft einen temperaturabhängigen Drahtwiderstand aufweist. Die Betriebssicherheit und Dauerhaltbarkeit des Sensors ist besonders hoch, wenn er vorzugsweise einen Thermistor aufweist.

Ebenfalls auf für die Niveauüberwachung gebräuchliche Sensoren kann man zurückgreifen, wenn gemäß einer anderen Weiterbildung des erfindungsgemäßen Verfahrens als Sensor ein kapazitiver Sensor zur Ermittlung von Änderungen der Dielektrizitätskonstante des den Sensor passierenden Ölstromes verwendet wird.

Eine ganz besonders vorteilhafte Weiterbildung des Verfahrens besteht darin, dass der Sensor in Höhe des minimal zulässigen Ölstandes einer Ölwanne des Verbrennungsmotors angeordnet wird und mit der Auswertelektronik zusätzlich zur Ermittlung von Blasen bei laufendem Verbrennungsmotor das Unterschreiten des minimalen Ölstandes in der Ölwanne bei stillstehendem Verbrennungsmotor ermittelt wird. Hierdurch kann der Sensor doppelt genutzt werden, nämlich einmal zur Überwachung der Motorschmierung bei laufendem und zum anderen zur Überwachung des Ölstandes bei stillstehendem Verbrennungsmotor.

Zur Ermittlung eines Füllstandes und des Vorhandenseins von Luftblasen ist es vorteilhaft, wenn durch den Widerstandskörper für jede Messung des Ölstandes ein festgelegtes Zeitintervall lang ein konstanter Strom fließengelassen wird und dabei der Spannungsabfall zu Beginn und am Ende der Bestromung bestimmt wird.

Zur fortlaufenden Überwachung eines Ölstromes hinsichtlich des Vorhandenseins von Blasen ist es vorteilhaft, wenn durch den Widerstandskörper für die kontinuierliche Detektion der Blasen ein konstanter Strom fließengelassen wird und dabei die Signalschwankungen der Spannung zur Feststellung der Gasblasenkonzentration bestimmt werden.

Für das erfindungsgemäße Verfahren kann man auch einen mechanisch arbeitenden Sensor einsetzen, wenn als Sensor ein mit einer vom Ölstrom auslenkbaren Stauklappe arbeitender Sensor verwendet wird. Hierbei macht man sich zunutze, dass durch Luftblasen im Ölstrom geringere Massenkräfte auf die Stauklappe wirken, so dass diese dann in unterschiedlichem Maß ausgelenkt wird.

Es genügt ein Sensor mit relativ geringer Ansprechgenauigkeit, wenn der Sensor oberhalb eines in etwa horizontal verlaufenden Ölleitungsbereiches der im oberen Bereich eines im unteren Bereich mit der Ölleitung in Verbindung stehenden Strömungstotraumes angeordnet ist. Hierdurch entsteht ein integrativer Effekt. Kleine, im Ölkreislauf auftretende Luftblasen, welche einzeln für sich kein Warnsignal auslösen würden, sammeln sich im Strömungstotraum. Hat sich dort so viel Luft angesammelt, dass der Sensor nicht mehr im Öl eingetaucht ist, löst dieser ein Warnsignal aus.

Die sich im Strömungstotraum sammelnde Luft strömt langsam zurück in den Ölkreislauf, so dass der Sensor bei nicht fortwährend auftretenden Luftblasen wieder in Öl eintaucht und dadurch funktionsfähig wird, wenn gemäß einer anderen Weiterbildung des Verfahrens der Strömungstotraum im unteren Bereich über einen großen Querschnitt mit der Ölleitung verbunden und sein oberer Bereich über einen kleineren Querschnitt mit der Ölleitung in einem Bereich geringeren Druckes verbunden ist.

Das zweitgenannte Problem, nämlich die Schaffung eines nach dem erfindungsgemäßen Verfahren arbeitenden Verbrennungsmotors, wird erfindungsgemäß dadurch gelöst, dass der Sensor und die Auswertelektronik zur Ermittlung von Gasblasen im Ölstrom der Ölleitung ausgebildet sind. Bei einem solchen Motor kann eine Warnung erfolgen, bevor überhaupt eine nicht ausreichende Ölschmierung erfolgt, sobald also ein solcher Zustand droht.

Eine besonders frühe Warnung erfolgt, wenn der Sensor in einer Ölansaugleitung in Strömungsrichtung vor einer Ölpumpe der Druckumlaufschmierung angeordnet ist.

Auch Undichtigkeiten im Bereich des Ölfilters und der Ölpumpe können detektiert werden, wenn der Sensor in Strömungsrichtung hinter der Ölpumpe und hinter einem der Ölpumpe nachgeschalteten Ölfilter angeordnet ist.

Besonders einfach ist der Sensor gestaltet, wenn er ein nach dem thermoelektrischen Prinzip arbeitender Sensor ist. Ein besonders einfacher und kostengünstiger Sensor liegt vor, wenn er einen temperaturabhängigen Drahtwiderstand aufweist. Der Sensor ist auch über einen großen Betriebszeitraum sehr zuverlässig, wenn er vorteilhaft einen Thermistor aufweist.

Vergleichbar einfach kann der Sensor ausgebildet sein, wenn der Sensor ein kapazitiver Sensor zur Ermittlung von Änderungen der Dielektrizitätskonstante des den Sensor passierenden Ölstromes ist.

Der Sensor vermag eine Doppelfunktion auszufüllen, wenn er in Höhe des minimal zulässigen Ölstandes einer Ölwanne des Verbrennungsmotors angeordnet und die Auswertelektronik zusätzlich zur Ermittlung von Blasen bei laufendem Verbrennungsmotor zur Ermittlung des Unterschreitens des minimalen Ölstandes in der Ölwanne bei stillstehendem Verbrennungsmotor ausgebildet ist.

Mit dem Sensor kann man nicht nur das Unterschreiten eines minimalen Ölstandes, sondern sogar den jeweiligen Ölstand messen, wenn gemäß einer anderen Weiterbildung der Erfindung der Sensor und die Auswertelektronik zur Ermittlung von Gasblasen im Ölstrom bei laufendem Verbrennungsmotor und zur Ermittlung des Ölstandes bei stillstehendem Verbrennungsmotor ausgebildet sind.

Bei dem Sensor kann es sich auch um ein mechanisch arbeitendes Bauteil handeln, wenn der Sensor eine vom Ölstrom auslenkbare Stauklappe aufweist.

Treten sehr feine, für den Sensor nicht feststellbare Luftblasen auf, so können diese zunächst gesammelt werden und bei Überschreiten einer festgelegten Luftblasenmenge ein Warnsignal erzeugen, wenn gemäß einer anderen Ausgestaltung der Erfindung der Sensor oberhalb eines in etwa horizontal verlaufenden Ölleitungsbereiches der Ölleitung im oberen Bereich eines im unteren Bereich mit der Ölleitung in Verbindung stehenden Strömungstotraumes angeordnet ist.

Die sich in dem Strömungstotraum sammelnde Luft wird automatisch zurück in den Ölkreislauf geführt, wenn der Strömungstotraum im unteren Bereich über einen großen Querschnitt mit der Ölleitung Verbindung hat und von seinem oberen Bereich eine Verbindung über einen kleineren Querschnitt mit der Ölleitung in einen Bereich geringeren Druckes besteht.

In beliebigen Querschnittsbereichen auftretende Luftblasen lassen sich durch den erfindungsgemäßen Sensor erfassen, wenn der Sensor in einer Höhe einen mäanderförmig über den Querschnitt der Ölleitung sich erstreckenden Widerstandsdraht hat.

Die Erfindung lässt verschiedene Ausführungsformen zu. Zur weiteren Verdeutlichung ihres Grundprinzips sind mehrere davon in der Zeichnung dargestellt und werden nachfolgend beschrieben. Diese zeigt in
- Fig. 1: eine Prinzipskizze einer Druckumlaufschmierung gemäß dem erfindungsgemäßen Verfahren,
- Fig. 2: ein nach dem thermoelektrischen Prinzip auf der Basis eines Widerstandsdrahtes arbeitender Sensor in einer Ölleitung,
- Fig. 3: ein nach dem thermoelektrischen Prinzip auf der Basis eines Thermistors arbeitender Sensor in einer Ölleitung,
- Fig. 4: ein mechanisch auf Basis einer Stauklappe arbeitender Sensor in einer Ölleitung,
- Fig. 5: ein nach dem kapazitiven Prinzip auf Zweiplattenbasis arbeitender Sensor in einer Ölleitung,
- Fig. 6: ein nach dem kapazitiven Prinzip auf Koaxialbasis arbeitender Sensor,
- Fig. 7: eine Anordnung eines Sensors in eine hierzu besonders gestaltete Ölleitung mit inteqrativem Effekt,
- Fig. 8: einen Querschnitt durch die Ölleitung im Bereich eines Sensors,
- Fig. 9: ein Diagramm, welches die Signalspannung eines Sensors über die Zeit für verschiedene Betriebszustände zeigt.

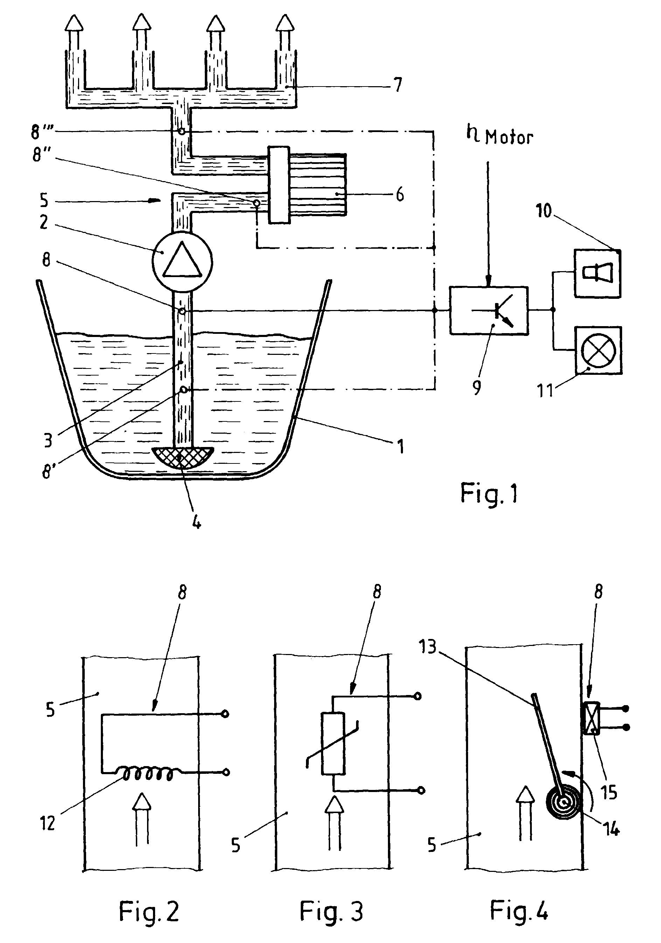

Die Figur 1 zeigt von einem Verbrennungsmotor eine Ölwanne 1, aus der mittels einer Ölpumpe 2 Öl gesaugt wird. Hierzu ist die Ölpumpe 2 in eine Ölleitung 5 geschaltet, welche mit einer senkrecht angeordneten Ölansaugleitung 3 mit einem nahe des Bodens der Ölwanne 1 angebrachten Ansaugfilter 4 in die Ölwanne 1 ragt. Von der Ölpumpe 2 führt die Ölleitung 5 zu einem Ölfilter 6 und von dort zu einer Ölverteilung 7.

In der Ölansaugleitung 3 ist ein Sensor 8 zur Überwachung von dort bei laufendem Verbrennungsmotor auftretenden Luftblasen angeordnet. Dieser Sensor 8 ist mit einer Auswertelektronik 9 verbunden, die zur Erzeugung eines Warnsignals einen Tongeber 10 oder eine Warnlampe 11 anzusteuern vermag. In Figur 1 sind strichpunktiert drei weitere Anbringungsmöglichkeiten des Sensors 8 dargestellt. Mit 8' ist die Anordnung eines Sensors in Höhe des minimalen Ölstandes, mit 8'' eine Anbringung unmittelbar vor dem Ölfilter 6 und mit 8''' eine Anbringung des Sensors vor der Ölverteilung positioniert. Ist der Sensor 8 an Position 8' in Höhe des minimal zulässigen Ölstandes angeordnet, so ergibt sich folgende Funktionserweiterung: Bestromt man den Sensor bei stillstehendem Motor, so kann man durch Ermittlung des Spannungsabfalls feststellen, ob er in Öl eingetaucht ist oder sich oberhalb des Ölspiegels in Luft befindet, weil er im letzteren Fall weniger gekühlt wird. Auf diese Weise kann man ein Signal für das Unterschreiten eines minimalen Ölstromes bei stillstehendem Verbrennungsmotor gewinnen.

Der in Figur 2 gezeigte Sensor 8 arbeitet nach dem thermoelektrischen Prinzip. Er hat einen als Draht ausgebildeten Widerstandskörper 12, welcher im Ölstrom der Ölleitung 5 angeordnet ist. Diesen Widerstandskörper 12 bestromt man periodisch mit konstantem Strom, wodurch sich der Widerstandskörper 12 erwärmt. Den dabei sich verändernden Spannungsabfall misst man. Treten im Ölstrom Gasblasen, insbesondere Luftblasen, auf, wird der Widerstandskörper 12 weniger gekühlt, was durch einen sich ändernden Spannungsabfall feststellbar ist. Alternativ kann zur kontinuierlichen Luftblasendetektion ein konstanter Strom durch den Widerstandskörper fließen und die Signalschwankungen der Spannung über dem Widerstandskörper zur Feststellung der Luftblasenkonzentration ausgewertet werden. Dies kann z. B. durch Bandpaßfilterung oder Mikroprozessor mit geeignetem Auswertealgorithmus geschehen.

Die Figur 3 zeigt einen Sensor 8 in der Ölleitung 5, der als Thermistor ausgebildet ist.

Der Sensor 8 gemäß Figur 4 hat eine Stauklappe 13, welche um eine horizontale Achse 14 schwenkbar angeordnet ist und die durch den Ölstrom in der Zeichnung gesehen nach oben bewegt wird. Ein Positionssensor 15 überwacht die Stellung der Stauklappe 13. Befinden sich im Ölstrom Luftblasen, dann wird die Stauklappe 13 weniger nach oben ausgelenkt, als das in Figur 4 zu sehen ist, so dass der Positionssensor 15 ein entsprechendes Signal zu erzeugen vermag.

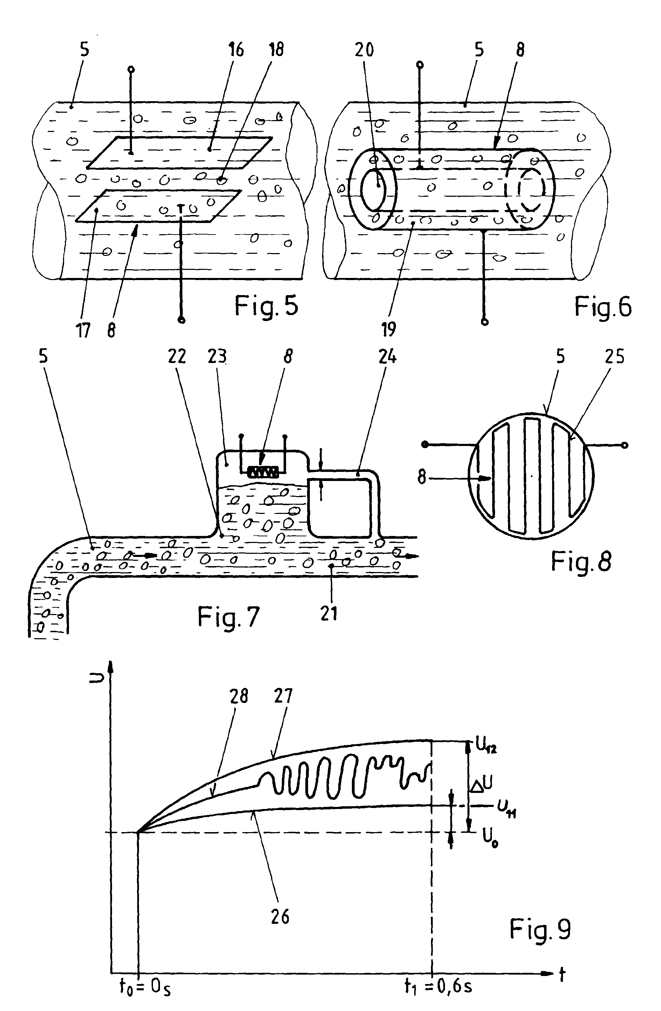

In den Figuren 5 und 6 sind die Sensoren 8 jeweils als Kondensator ausgebildet. Figur 5 zeigt zwei Kondensatorplatten 16, 17, zwischen denen sich ein Teilbereich des Ölstromes bewegt. Sind in dem Ölstrom Luftblasen 18 vorhanden, ändert sich die Dielektrizitätskonstante des Mediums, was zu einer Kapazitätsänderung des Kondensators führt.

Die Ausführungsform nach Figur 6 unterscheidet sich lediglich baulich von der nach Figur 5, indem die beiden Kondensatorplatten 16, 17 durch zwei koaxiale Rohre 19, 20 ersetzt wurden.

Die Figur 7 zeigt einen horizontal verlaufenden Ölleitungsbereich 21 mit einem nach oben gerichteten, topfförmigen Ansatz 22, durch welchen ein Strömungstotraum 23 gebildet ist. Oben in diesem Strömungstotraum 23 ist der Sensor 8 angeordnet. Von dem oberen Bereich des Strömungstotraumes 23 führt eine Verbindung 24 zurück in den Ölleitungsbereich 21 zu einer Stelle, welche geringeren Druck hat als im Bereich des Ansatzes 22. Diese Verbindung 24 hat geringeren Querschnitt als der Ansatz 22. Dadurch sammelt sich im Ansatz 22 schneller Luft, als sie ihn wieder verlassen kann, so dass bei einer größeren Anzahl von Luftblasen innerhalb einer kurzen Zeit der Sensor 8 sich außerhalb des Öls befindet, deshalb wärmer wird und ein Warnsignal auslöst.

Bei der Ausführungsform nach Figur 8 ist der Sensor 8 durch einen Widerstandsdraht 25 gebildet, der über den gesamten Querschnitt der Ölleitung 5 mäanderförmig verläuft. Dadurch wird sichergestellt, dass ein hoher Anteil der im Ölstrom auftretenden Luftblasen gegen den Widerstandsdraht 25 gelangen und auf diese Weise den Sensor 8 beeinflussen.

Das Diagramm gemäß Figur 9 zeigt die Signalspannung eines nach dem thermoelektrischen Prinzip arbeitenden Sensors 8 über die Zeit. Wird der Sensor 8 zur Niveaumessung benutzt und ist er vollständig in Öl eingetaucht, dann entsteht eine untere Kurve 26. Zum Zeitpunkt t₀ beginnt die Bestromung des Sensors 8. Dadurch erhöht sich die Temperatur seines Widerstandsdrahtes. Dieser Anstieg ist jedoch relativ gering, so dass auch der Spannungsabfall über den Widerstandsdraht bis zum Ende der Bestromung zum Zeitpunkt t₁ (Spannung U₁₁) nur relativ geringfügig zunimmt.

Befindet sich der Sensor 8 außerhalb des Öles, dann wird er weniger gekühlt und deshalb wärmer. Die obere Kurve 27 zeigt, dass dann ein größerer Spannungsabfall (vgl. Spannung U₁₂ zum Zeitpunkt t₁) auftritt, aus dem auf diesen Zustand geschlossen werden kann. Um den Einfluss der Öltemperatur zu kompensieren, misst man jeweils zunächst den Spannungsabfall zu Beginn der Bestromung und vergleicht diesen mit dem Spannungsabfall während der Dauer der Bestromung und vor allem am Ende der Bestromung.

Die mittlere Kurve 28 zeigt, dass während des Betriebs des Verbrennungsmotors beim Auftreten von Luftblasen die Spannung sehr plötzlich zwischen höheren und tieferen Werten schwankt. Diese raschen Schwankungen können beispielsweise durch Bandpassfilter erkannt und in der Auswertelektronik 9 erfasst werden, so dass aus diesen Schwankungen auf das Vorhandensein von Luftblasen im Ölstrom geschlossen werden kann.

## Patentansprüche

1. Verfahren zur Überwachung der ausreichenden Ölschmierung eines Verbrennungsmotors, insbesondere eines Fahrzeugs, mittels eines in einer Ölleitung einer Druckumlaufschmierung angeordneten, mit einer Auswertelektronik verbundenen Sensors, **dadurch gekennzeichnet,** dass mittels des Sensors (8) und der Auswertelektronik (9) Gasblasen (18) im Ölstrom ermittelt werden.

2. Verfahren nach Anspruch 1, **dadurch gekennzeichnet,** dass der Sensor (8, 8') in einer Ölansaugleitung (3) in Strömungsrichtung vor einer Ölpumpe (2) der Druckumlaufschmierung angeordnet wird.

3. Verfahren nach Anspruch 1, **dadurch gekennzeichnet,** dass der Sensor (8, 8''') in Strömungsrichtung hinter der Ölpumpe (2) und hinter einem der Ölpumpe (2) nachgeschalteten Ölfilter (6) angeordnet wird.

4. Verfahren nach einem der vorangehenden Ansprüche, **dadurch gekennzeichnet,** dass als Sensor (8) ein nach dem thermoelektrischen Prinzip arbeitender Sensor verwendet wird.

5. Verfahren nach Anspruch 4**, dadurch gekennzeichnet,** dass der Sensor (8) einen temperaturabhängigen Drahtwiderstand aufweist.

6. Verfahren nach Anspruch 4, **dadurch gekennzeichnet,** dass der Sensor (8) einen Thermistor aufweist.

7. Verfahren nach einem der Ansprüche 1 bis 3, **dadurch gekennzeichnet,** dass als Sensor (8) ein kapazitiver Sensor (16, 17; 19, 20) zur Ermittlung von Änderungen der Dielektrizitätskonstante des den Sensor passierenden Ölstromes verwendet wird.

8. Verfahren nach einem der vorangehenden Ansprüche, **dadurch gekennzeichnet,** dass der Sensor (8, 8') in Höhe des minimal zulässigen Ölstandes einer Ölwanne (1) des Verbrennungsmotors angeordnet wird und mit der Auswertelektronik (9) zusätzlich zur Ermittlung von Blasen (18) bei laufendem Verbrennungsmotor das Unterschreiten des minimalen Ölstandes in der Ölwanne (1) bei stillstehendem Verbrennungsmotor ermittelt wird.

9. Verfahren nach Anspruch 8, bei dem der Widerstandskörper des Sensors sich in der Ansaugleitung in etwa in Vertikalrichtung erstreckt**, dadurch gekennzeichnet,** dass durch den Widerstandskörper für jede Messung des Ölstandes ein festgelegtes Zeitintervall lang ein konstanter Strom fließengelassen wird und dabei der Spannungsabfall zu Beginn und am Ende der Bestromung bestimmt wird.

10. Verfahren nach Anspruch 8, **dadurch gekennzeichnet,** dass durch den Widerstandskörper für die kontinuierliche Detektion der Blasen ein konstanter Strom fließengelassen wird und dabei die Signalschwankungen der Spannung zur Feststellung der Gasblasenkonzentration bestimmt werden.

11. Verfahren nach einem der vorangehenden Ansprüche, **dadurch gekennzeichnet,** dass als Sensor (8) ein mit einer vom Ölstrom auslenkbaren Stauklappe (13) arbeitender Sensor verwendet wird.

12. Verfahren nach einem der vorangehenden Ansprüche, **dadurch gekennzeichnet,** dass der Sensor oberhalb eines in etwa horizontal verlaufenden Olleitungsbereiches (21) im oberen Bereich eines im unteren Bereich mit der Ölleitung in Verbindung stehenden Strömungstotraumes (23) angeordnet ist.

13. Verfahren nach Anspruch 12**, dadurch gekennzeichnet,** dass der Strömungstotraum (23) im unteren Bereich über einen großen Querschnitt mit der Ölleitung verbunden und sein oberer Bereich über einen kleineren Querschnitt mit der Ölleitung in einem Bereich geringeren Druckes verbunden ist.

14. Verbrennungsmotor zur Durchführung des Verfahrens nach einem der vorangehenden Ansprüche, mit einer Vorrichtung zur Überwachung der ausreichenden Ölschmierung mittels eines in einer Ölleitung einer Druckumlaufschmierung angeordneten, mit einer Auswertelektronik verbundenen Sensors, **dadurch gekennzeichnet,** dass der Sensor (8) und die Auswertelektronik (9) zur Ermittlung von Gasblasen im Ölstrom ausgebildet sind.

15. Verbrennungsmotor nach Anspruch 12, **dadurch gekennzeichnet,** dass der Sensor (8, 8') in einer Ölansaugleitung (3)in Strömungsrichtung vor einer Ölpumpe (2) der Druckumlaufschmierung angeordnet ist.

16. Verbrennungsmotor nach Anspruch 14, **dadurch gekennzeichnet,** dass der Sensor (8', 8''') in Strömungsrichtung hinter der Ölpumpe (2) und hinter einem der Ölpumpe (2) nachgeschalteten Ölfilter (6) angeordnet ist.

17. Verbrennungsmotor nach einem der Ansprüche 14 bis 16, **dadurch gekennzeichnet,** dass der Sensor (8) ein nach dem thermoelektrischen Prinzip arbeitender Sensor ist.

18. Verbrennungsmotor nach Anspruch 17, **dadurch gekennzeichnet,** dass der Sensor (8) einen temperaturabhängigen Drahtwiderstand aufweist.

19. Verbrennungsmotor nach Anspruch 17, **dadurch gekennzeichnet,** dass der Sensor (8) eine Thermistor aufweist.

20. Verbrennungsmotor nach einem der Ansprüche 14 bis 16, **dadurch gekennzeichnet,** dass der Sensor (8) ein kapazitiver Sensor (16, 17; 19, 20) zur Ermittlung von Änderungen der Dielektrizitätskonstante des den Sensor (8) passierenden Ölstromes ist.

21. Verbrennungsmotor nach einem der vorangehenden Ansprüche, **dadurch gekennzeichnet,** dass der Sensor (8, 8') in Höhe des minimal zulässigen Ölstandes einer Ölwanne (1) des Verbrennungsmotors angeordnet und die Auswertelektronik (9) zusätzlich zur Ermittlung von Blasen bei laufendem Verbrennungsmotor zur Ermittlung des Unterschreitens des minimalen Ölstandes in der Ölwanne (1) bei stillstehendem Verbrennungsmotor ausgebildet ist.

22. Verbrennungsmotor nach Anspruch 21, bei dem der Widerstandskörper des Sensors sich in der Ansaugleitung in etwa in Vertikalrichtung erstreckt, **dadurch gekennzeichnet,** dass der Sensor (8) und die Auswertelektronik (9) zur Ermittlung von Gasblasen im Ölstrom bei laufendem Verbrennungsmotor und zur Ermittlung des Ölstandes bei stillstehendem Verbrennungsmotor ausgebildet sind.

23. Verbrennungsmotor nach Anspruch 11, **dadurch gekennzeichnet,** dass der Sensor (8) eine vom Ölstrom auslenkbare Stauklappe (13) aufweist.

24. Verbrennungsmotor nach einem der vorangehenden Ansprüche, **dadurch gekennzeichnet,** dass der Sensor (8) oberhalb eines in etwa horizontal verlaufenden Ölleitungsbereichs (21) der Ölleitung (5) im oberen Bereich eines im unteren Bereich mit der Ölleitung (in Verbindung stehenden Strömungstotraumes (23) angeordnet ist.

25. Verbrennungsmotor nach Anspruch 24, **dadurch gekennzeichnet,** dass der Strömungstotraum (23) im unteren Bereich über einen großen Querschnitt mit der Ölleitung Verbindung hat und von seinem oberen Bereich eine Verbindung (24) über einen kleineren Querschnitt mit der Ölleitung (5) in einen Bereich geringeren Druckes besteht.

26. Verbrennungsmotor nach einem der vorangehenden Ansprüche, **dadurch gekennzeichnet,** dass der Sensor (8) in einer Höhe einen mäanderförmig über den Querschnitt der Ölleitung (5) sich erstreckenden Widerstandsdraht (25) hat.
